# EUROPÄISCHE PATENTSCHRIFT

(11) **EP 3 384 369 B1**
(45) Veröffentlichungstag und Bekanntmachung des Hinweises auf die Patenterteilung: **01.03.2023**
(21) Anmeldenummer: 16805145.6
(22) Anmeldetag: 02.12.2016
(51) Int. Cl.: G06F 3/041, G16H 40/20, G16H 40/63

(54) **DESINFEKTIONSSTATUSÜBERWACHUNG FÜR DIALYSEMONITORE**
DISINFECTION STATUS MONITORING FOR DIALYSIS MONITORS
CONTRÔLE DE L'ÉTAT DE DÉSINFECTION POUR DES MONITEURS DE DIALYSE

(30) Priorität: 03.12.2015 DE 102015121014
(43) Veröffentlichungstag der Anmeldung: 10.10.2018
(73) Patentinhaber: Fresenius Medical Care Deutschland GmbH, 61352 Bad Homburg (DE)
(72) Erfinder: WUEPPER, Andreas, 64572 Buettelborn (DE); HEIDE, Alexander, 65817 Eppstein (DE); NIKOLIC, Dejan, 65812 Bad Soden (DE)
(74) Vertreter: Schwarz, Claudia
(86) Internationale Anmeldenummer: PCT/EP2016/079588
(87) Internationale Veröffentlichungsnummer: WO 2017/093484

(56) Entgegenhaltungen:
- EP-A1- 2 908 237
- US-A1- 2011 116 967
- US-A1- 2015 258 228

## Beschreibung

Die vorliegende Erfindung betrifft die Überwachung eines Desinfektionsvorganges bzw. einer Desinfektion von berührungssensitiven Monitoren eines Dialysegerätes oder anderer medizinischer Geräte. Sie bezieht sich insbesondere auf eine Desinfektionsüberwachungseinheit, auf ein Touchscreen, auf ein medizinisches Gerät, auf ein System und auf ein Verfahren zur Steuerung eines Dialysegerätes oder eines anderen medizinischen Gerätes.

Bekannte, moderne medizintechnische Geräte umfassen häufig einen berührungssensitiven Bildschirm, einen Touchscreen, zur Steuerung des Gerätes und zur Ein- und Ausgabe von Daten, wie beispielsweise das Hämodialysegerät 5008 von Fresenius Medical Care.

Für die Bedienoberflächen der Dialysegeräte wird üblicherweise eine kapazitive Sensortechnik verwendet. Ein Touchscreen mit einer kapazitiven Sensortechnik ist beispielsweise in der DE10 2011 011 769 A1 näher beschrieben, bei der eine zusätzliche piezoelektrische Schicht mit Piezoelementen verwendet wird, um taktiles Feedback zu ermöglichen.

Aus dem Stand der Technik sind Verfahren zur Sterilisation oder zur Desinfektion von Geräteoberflächen bekannt, wie die US 2015/0258228 A1, die US 2011/0116967 A1 sowie die EP 2 908 237 A1.

Die Dialysegeräte, insbesondere deren Touchscreensensoren zur Bedienung und Steuerung des Gerätes, werden in der klinischen Praxis in der Regel von mehreren Personen benutzt oder von einer Person, die jedoch in Sequenz mehrere medizinische und potentiell infizierte Geräte bedient und zwischen den Bedienungen nicht immer eine Desinfektion ausführt. Deshalb kann es über die Berührung des Bildschirms zu ungewollten Verunreinigungen kommen, indem z.B. pathogene Keime oder Mikroorganismen eine Kreuzkontamination verursachen, also eine direkte oder indirekte Übertragung von potentiell schädlichen Substanzen.

Um eine solche Übertragung von Krankheitserregern und Verunreinigungen zu vermeiden, ist es im Stand der Technik bekannt, nach dem Aufrüsten der Maschine bzw. des Dialysegerätes und nach dem Start der Behandlung den Touchscreensensor routinemäßig zu desinfizieren. Auch während des Betriebs des Dialysegerätes ist eine Desinfektion vorgesehen. Dazu wird ein Schalter am Dialysegerät oder am Monitor betätigt, der den Touchscreensensor in einen "insensitiven" Modus schaltet, so dass der Touchscreensensor vorübergehend deaktiviert wird, um nicht intendierte Steuermaßnahmen (z.B. durch ungewolltes Betätigen einer Schaltfläche) zu vermeiden. Nach der Desinfektion kann der Touchscreensensor dann wieder auf "sensitiv" geschaltet und zur Gerätesteuerung verwendet werden.

Das Problem bei den bekannten Geräten aus dem Stand der Technik ist darin zu sehen, dass es nicht erkennbar ist, in welchem Zustand sich der Touchscreensensor befindet. Insbesondere ist es unklar, ob der Touchscreensensor und all seine Segmente ausreichend und korrekt desinfiziert sind. Dies führt zum einen zur Gefahr von Kreuzkontaminationen und zum anderen dazu, dass Desinfektionsmaßnahmen möglicherweise nicht in ausreichender Qualität durchgeführt werden und deshalb wiederholt werden müssen, was zu einer Zeitverzögerung führt.

Die vorliegende Erfindung hat sich deshalb zur Aufgabe gestellt, die Bedienoberfläche von medizinischen Geräten, insbesondere von Dialysemaschinen, zu verbessern. Insbesondere soll die Sicherheit bei der Bedienung von Dialysemaschinen erhöht und die Gefahr von Kreuzkontaminationen verringert werden. Ferner soll der Prozess der Desinfektion verbessert werden, insbesondere was die Qualität der Desinfektion betrifft.

Diese Aufgabe wird erfindungsgemäß durch eine Desinfektionsüberwachungseinheit, einen Touchscreen mit einer solchen Desinfektionsüberwachungseinheit, einem medizinischen Gerät und einem Verfahren gemäß den beiliegenden nebengeordneten Patentansprüchen gelöst.

Im Folgenden wird die Erfindung anhand der vorrichtungsgemäßen Aufgabenlösung, und somit u.a. anhand der Desinfektionsüberwachungseinheit beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf ein System oder auf eine Vorrichtung gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch elektronische Hardware-Module oder Mikroprozessor-Module, des Systems bzw. des Produktes ausgebildet und umgekehrt.

Die erfindungsgemäße Desinfektionsüberwachungseinheit ist für einen Touchscreensensor eines Touchscreens zur Steuerung eines medizinischen Gerätes, insbesondere eines Dialysegerätes, ausgelegt. Die Desinfektionsüberwachungseinheit kennzeichnet sich dadurch, dass sie einen Desinfektionszustand des Touchscreensensors oder Segmenten des Touchscreensensors erfasst.

Bei dem Touchscreen handelt es sich in einer bevorzugten Ausführungsform der Erfindung um einen kapazitiven Touchscreen mit Multi-Sensor-Funktionalität, der auch gleichzeitige Berührungen detektieren kann. Ein Beispiel eines solchen Touchscreens ist in der DE10 2011 011 769 A1 näher beschrieben. Üblicherweise umfasst der Touchscreen einen Touchscreensensor als eigentliche Eingabeeinheit, einen Controller, der in oder am Touchscreensensor angeordnet sein kann und fakultativ einen Treiber, der in dem medizinischen Gerät angeordnet sein kann. In einer alternativen und ebenfalls bevorzugten Ausführungsform der Erfindung kann der Touchscreensensor als projiziert-kapazitiver Sensor ausgebildet sein (meistens "PCT" = "Projected Capacitive Touch" oder "PCAP" genannt). Dabei nutzt der Sensor zwei Ebenen mit einem leitfähigen Muster (beispielsweise Streifen oder Rauten). Die Ebenen sind voneinander isoliert angebracht. Befindet sich ein Finger am Kreuzungspunkt zweier Streifen, so ändert sich die Kapazität des Kondensators, und es kommt ein größeres Signal am Empfängerstreifen an. Diese Signaländerung lässt sich somit genau anhand der X-und Y-Koordinaten messen, wobei auch mehrere Berührungspunkte exakt definierbar sind. Der Stromfluss von den Ecken des Touchscreens zum Berührungspunkt ist proportional zu den XY-Koordinaten. Der wesentliche Vorteil dieses Systems ist, dass der Sensor auf der Rückseite des Deckglases angebracht werden kann, da die Berührungserkennung durch das Glas "hindurchprojiziert" wird. So erfolgt die Bedienung auf der praktisch verschleißfreien Glasoberfläche. Ferner ist die Erkennung von Gesten und mehreren Berührungen (also Multi-Touch) möglich. In anderen Ausführungsform der Erfindung können jedoch auch resistive oder induktive oder andere Sensortechnologien für Touchscreens zur Anwendung kommen.

Der Touchscreen stellt eine Benutzeroberfläche (auch Monitor oder Display genannt) dar und umfasst den Sensor (Touchscreensensor) zur Erfassung der Eingabesignale (z.B. auf Eingabefeldern) zur Steuerung und Bedienung des Dialysegerätes (im Folgenden auch Gerät oder Maschine genannt) sowie ein Display, auf dem Interaktionsflächen, Schaltelemente, Kontrollfelder etc. zur Steuerung des Dialysegerätes dargestellt sein können. Üblicherweise ist der Tochscreen so aufgebaut, dass sein Display und der Touchscreensensor beide auf dem gesamten Bereich des Touchscreens lokalisiert sind und sich insbesondere vollständig überdecken. Auch wenn dies für die Ausbildung der Erfindung nicht zwangsläufig der Fall sein muss, so wird im Folgenden davon ausgegangen, dass die Fläche des Touchscreens mit der Fläche seines Touchscreensensors übereinstimmt, wobei der Touchscreen und der Touchscreensensor bündig übereinander angeordnet sind. Der Anwender differenziert in der Regel nicht zwischen dem Touchscreen und dem Touchscreensensor und nimmt die beiden technischen Systeme als eine Einheit wahr. Mit anderen Worten ist der Touchscreensensor die oberste, berührbare Schicht des Touchscreens. Insofern wird bei der Erfassung des Desinfektionsstatus' des Touchscreensensors zugleich der Desinfektionsstatus des Touchscreens erfasst und gegebenenfalls verrechnet, um weitere Maßnahmen anzusteuern, und umgekehrt. Grundsätzlich soll für alle Berührungen auf dem Touchscreen zur Steuerung des Gerätes der Desinfektionszustand erfasst und überprüft und gegebenenfalls ausgegeben werden, z.B. in Form eines Symbols auf dem Touchscreen (z.B. eine grüne Flagge bei einem desinfizierten Zustand und eine rote Flagge andernfalls oder bei einem nicht-desinfizierten Zustand). Der Desinfektionszustand wird dabei auf der berührbaren Oberfläche des Touchscreensensors erfasst.

Im Folgenden wird die Erfindung für ein Dialysegerät als Beispiel eines medizinischen Gerätes beschrieben, z.B. eines Hämodialysegerätes oder eines Peritonealdialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die über einen Touchscreen gesteuert werden.

Der Desinfektionsstatussensor dient zur Erfassung des Desinfektionszustandes des Touchscreensensors oder Segmenten des Touchscreensensors und kann als separate bauliche Einheit ausgebildet sein, die über entsprechende drahtgebundene oder drahtlose Schnittstellen zum Datenaustausch mit dem Touchscreen verbunden ist, insbesondere, wenn der Desinfektionsstatussensor dazu ausgebildet ist, den Desinfektionszustand optisch (z.B. über einen Kamerasensor oder über einen weiteren Monitor) zu erfassen. Vorzugsweise ist der Desinfektionsstatussensor jedoch in den Touchscreen integriert, insbesondere, wenn die Detektion der Desinfektion über den Bedienungsmodus (also über eine Auswertung der Bedienkontakte) des Touchscreensensors erfolgt (automatisches Erfassen von flächigen Wischbewegungen).

Der Begriff "Desinfektionszustand" bezieht sich auf den Zustand des Touchscreens mit dem Touchscreensensor und damit auf die berührbare Bedienoberfläche des Touchscreens (bzw. Monitors) zur Steuerung des Dialysegerätes. Im Wesentlichen sind zwei Zustandsgrößen vorgesehen: 1. Desinfiziert (also keimfrei) und 2. Nicht desinfiziert oder nicht ausreichend desinfiziert mit der Gefahr von Kreuzkontaminationen. Letzterer Zustand kann z.B. auch dann eintreten und erfasst werden, wenn nicht alle Segmente des Touchscreens gereinigt worden sind oder wenn die Reinigung/Desinfektion zu kurz war oder schon zu lange her ist. Der Desinfektionszustand kann mehrere Zustandsgrößen umfassen, wobei die jeweiligen Zustandsgrößen den jeweiligen Desinfektionszustand auf einzelnen separaten und vorzugsweise nicht-überlappenden Segmenten des Touchscreensensors kennzeichnen. Damit wird es möglich, auch unterschiedliche Segmente des Touchscreensensors zu analysieren, um so - was die Ortsauflösung betrifft - eine genauere und feiner granulierte Aussage zu ermöglichen. Somit kann beispielsweise erfasst werden, dass bestimmte Segmente desinfiziert sind, während andere Segmente sich (noch) in einem nicht-desinfizierten Zustand befinden.

Ein wesentlicher Gedanke der Erfindung ist darin zu sehen, dass der Touchscreensensor qualitativ zuverlässig, kontinuierlich und gemäß vorbestimmbaren Bedingungen (u.a. hinsichtlich Zeit, Dauer, Umfang und Qualität) auf die Gefahr von Kontaminationen analysiert wird und dass das Analyseergebnis in Form des Desinfektionszustandes ausgegeben wird. Damit kann vorteilhafterweise auch der Desinfektionsprozess verbessert werden, indem Zusatzinformationen und relevante Informationen zur Desinfektion (z.B. Anzeige als desinfiziert erkannter Segmente und als nicht-desinfiziert erkannter Segmente) als Unterstützung bereitgestellt werden.

Gemäß der Erfindung umfasst die Desinfektionsüberwachungseinheit einen Desinfektionsstatussensor, der zur automatischen Erfassung des Desinfektionsstatus ausgebildet ist. Dies kann über eine Analyse der Bedienkontakte auf dem Touchscreensensor erfolgen (Punkt/Drückkontakte im Unterschied zu Wischkontakten) und/oder über eine Analyse der kontaktierten Flächenbereiche des Touchscreensensors. Erfindungsgemäß wird das Vorhandensein von Desinfektionsmittel, in der Regel von Desinfektionsflüssigkeit, überprüft. Alternativ kann der Desinfektionszustand auch manuell erfasst werden, also ohne Sensor, indem der Anwender (in der Regel die Krankenschwester) nach erfolgter Desinfektion einen Schalter am Dialysegerät oder am Touchscreen oder am jeweiligen Bedienelement betätigt. Bei Betätigen der Taste oder des Schalters gilt die Desinfektion als erfolgt und wird entsprechend als Desinfektionsstatus bzw. Desinfektionszustand erfasst.

In einer bevorzugten Ausführungsform der Erfindung ist der erfasste Desinfektionszustand als Zustandsvektor repräsentiert. Er umfasst in der Regel mehrere Zustandsgrößen, die für die Desinfektion des Touchscreensensors relevant sind. Die Zustandsgrößen, die von der Desinfektionsüberwachungseinheit erfasst und berechnet werden, können in einer bevorzugten Ausführungsform der Erfindung in einer Definitionsphase konfiguriert werden. Der Zustandsvektor kann insbesondere kennzeichnen, ob eine Desinfektion stattgefunden hat. Weiterhin kann er kennzeichnen, welche Qualität die bisherige Desinfektion hat. Dies wird möglich, indem erfasst wird, in welchem Bereich der Touchscreensensor desinfiziert wurde und - gegebenenfalls - wie lange und wann die Desinfektion erfolgt ist. Dazu kann der Zustandsvektor eine Bereichsangabe umfassen, die den Umfang der Desinfektion kennzeichnet. Gemäß einem weiteren Aspekt der Erfindung kann der Zustandsvektor noch weitere Meta-Daten umfassen, die im Rahmen der Desinfektion relevant sind. Diese Meta-Daten umfassen: Zeitparameter, die den Zeitpunkt und/oder die Dauer der Desinfektion kennzeichnen, die Verwendung von Desinfektionsmittels in ausreichendem Maße, den Umfang der Desinfektion hinsichtlich der desinfizierten Fläche des Touchscreens oder des jeweiligen Touchscreensegmentes etc.

In einer vorteilhaften Weiterbildung der Erfindung umfasst die Desinfektionsüberwachungseinheit eine Ausgabeeinheit, die dazu ausgebildet ist, den erfassten Desinfektionszustand als Desinfektionssignal oder Desinfektionsnachricht auszugeben. Die Ausgabe kann entweder unmittelbar auf der Benutzeroberfläche des Dialysegerätes, also des Touchscreens, erfolgen oder mittelbar auf andere elektronischen Instanzen, die mit der Desinfektionsüberwachungseinheit in Datenaustausch stehen (z.B. auf einem angeschlossenen akustischen und/oder optischen Signalgeber). Vorzugsweise wird der erfasste Desinfektionszustand direkt und unmittelbar auf dem Touchscreen ausgegeben werden, z.B. in Form eines Symbols, das den Zustand repräsentiert (z.B. in Form einer grünen oder roten Flagge). Dabei zielt eine bevorzugten Ausführungsform der Erfindung darauf ab, mehrere Symbole für die jeweiligen Bedienfelder auf der Benutzeroberfläche darzustellen, um eine spezifizierte Zustandserfassung für die einzelnen Touchscreensegmente auf einen Blick zu ermöglichen.

Alternativ kann der erfasste Desinfektionszustand als Berechnungsergebnis an weitere Instanzen weitergeleitet werden, um weitere technische Maßnahmen zu triggern, wie z.B. das Ausgeben an eine Aufforderungseinheit zur Ausgabe eines Aufforderungssignals zur Desinfektion und/oder zur Ausgabe an einen Warnsignalgeber zur Ausgabe eines Warnsignals, um auf eine nicht erfolgte Desinfektion oder eine Desinfektion in nicht ausreichender Qualität hinzuweisen, da z.B. erfasst worden ist, dass nicht der vollständige Bereich des Touchscreensensors in ausreichendem Maße desinfiziert worden ist. Gemäß einem Aspekt kann es im Vorfeld konfiguriert werden, in welchem Format der erfasste Desinfektionszustand ausgegeben wird. So kann er visuell in einem Textformat als Nachricht angezeigt werden oder akustisch über einen Lautsprecher in Form einer akustischen gesprochenen Nachricht ausgegeben werden. Weiterhin kann in einer sehr einfachen Ausführungsform der Erfindung nur ein akustisches Warnsignal ausgegeben werden, das signalisiert, dass eine Desinfektion notwendig ist. Hier kann ein weiteres Sicherheitsmodul angesteuert werden, das dazu bestimmt ist, bei einer als nicht ausreichend erkannte Desinfektion das Warnsignal auszugeben. Ebenso ist es möglich, das Warnsignal und/oder den erfassten Desinfektionszustand an andere Geräte (z.B. dem medizinischen Gerät selbst) weiterzuleiten und dort zur Ausgabe zu bringen oder weitere technische Maßnahmen auf dem Sicherheitsmodul auszuführen (z.B. einen modifizierten Bildschirmaufbau zu steuern).

In einer anderen, vorteilhaften Weiterbildung der Erfindung ist der Desinfektionsstatussensor dazu ausgebildet, den Desinfektionszustand über eine Identifikation von zeitlich vorhergehenden Bewegungsmustern auf dem Touchscreensensor zu erfassen. Erfindungsgemäß werden flächige Bewegungsmuster (z.B. flächige Wischbewegungen) zur Desinfektion automatisch erkannt und von Punkt- oder Druckbedienungen zur Eingabe von Daten bzw. zur Gerätesteuerung differenziert. Erfindungsgemäß ist auch ein separater optischer Sensor, beispielsweise eine Kamera, vorgesehen, der die Bedienungen oder Kontakte des Touchscreensensors erfasst und an die Desinfektionsüberwachungseinheit zur Auswertung weiterleitet. Die Kamera kann an einer Seite des Touchscreensensors angeordnet sein, um zu erfassen, wo die Desinfektion ausgeführt worden ist und insbesondere welche Segmente des Touchscreensensors durch ein Tuch abgewischt worden sind. Dazu könnte beispielsweise der in der DE10 2011 011 767 A1 beschriebene optische Sensor verwendet werden. Ferner können Infrarotsensoren zur Erfassung der Desinfektion über eine Wärmeentwicklung eingesetzt werden. Ebenso ist es möglich, das Vorhandensein von Desinfektionsmittel auf dem Touchscreen zu erfassen. Bei dieser Art der Sensorik wird der technische Effekt ausgenutzt, dass das Wischtuch durch die Benetzung des Touchscreensensors mit Desinfektionsflüssigkeit feucht wird und somit leitfähig (die Eigenleitfähigkeit des Desinfektionsmittel vorausgesetzt), und somit einen Effekt auf die Sensorik des Touchscreensensors hat. Da auch hier ein Ladungstransport über die menschliche Hand notwendig ist, muss sichergestellt werden, dass das feuchte Wischtuch mit der Hand in Berührung kommt.

Die Art des Desinfektionsprozesses ist nicht auf ein Abwischen des Touchscreensensors mit einem Tuch mit Desinfektionsmittel beschränkt, sondern es können auch spezielle Desinfektionsvorrichtungen, wie Abzieher (vergleichbar einem Abzieher beim Fensterputzen) oder Sprühvorrichtungen oder dergleichen zum Einsatz kommen. Derartige Vorrichtungen könnten auch bei resistiven oder induktiven Touchscreen-Displays verwendet werden, wenn sie derart ausgestaltet sind, dass mit ihnen eine sichere Erfassung der Berührung möglich ist.

Der jeweilige Desinfektionsstatussensor (je nach Ausführungsform der Erfindung: Kamera, Auswerteeinheit für die Kontakte auf der Oberfläche des Touchscreensensors oder Fluidauswertung etc.) kann gesteuert aktiviert werden, um zu vermeiden, dass er nicht unnötig Daten erfasst, sondern nur während einer Desinfektionsphase und nicht während der Gerätesteuerung. Dazu kann ein Schaltelement in der Desinfektionsüberwachungseinheit bereitgestellt werden, das unmittelbar vor einer auszuführenden Desinfektion aktiviert wird. Nur bei Aktivierung des Schaltelementes wird dann der Desinfektionsstatussensor aktiviert. Damit kann sowohl Energie eingespart als auch unnötige Erfassung und Speicherung von Daten vermieden werden, was insgesamt die Ressourcen schont.

Gemäß einer Variante ist der Desinfektionsstatussensor ausgebildet, den Desinfektionszustand von mehreren (verschiedenen und vorzugsweise nicht-überlappenden) vordefinierten Segmenten des Touchscreensensors zu erfassen. Dies erweist sich insbesondere dann als vorteilhaft, wenn ein oder mehrere Anwender den Touchscreensensor in unterschiedlichen Segmenten benutzen und somit die Gefahr von Kreuzkontaminationen nur in bestimmten Segmenten des Touchscreens besteht.

Grundsätzlich kann der Desinfektionszustand gemäß einer bevorzugten Ausführung der Erfindung spezifisch für den jeweiligen Anwender erfasst und angezeigt werden. Dazu kann vorzugsweise automatisch die Nutzeridentität erfasst werden. Deshalb wird in einer vorteilhaften Weiterbildung automatisch oder auf ein Bestätigungssignal hin die Nutzeridentität der jeweiligen Bedienperson des Touchscreensensors erfasst. Die Nutzeridentität ist jeweils spezifisch für einen Anwender des Touchscreensensors. Sobald ein Nutzerwechsel erfasst wird, wird das Ausgeben eines Aufforderungssignals zur Desinfizierung des Touchscreens ausgelöst. Falls auf ein Aufforderungssignal hin keine anschließende Desinfektion erkannt wird, kann es vorgesehen sein, automatisch einen Gefährdungs-Warnhinweis auszugeben, um Kreuzkontaminationen zu vermeiden. Die Nutzeridentität kann über die Eingabe der Nutzerkennung oder eines Passwortes erfolgen, wie z.B. über die Eingabe von biometrischen Signalen, Nutzerkarten mit Radio-Frequenz-Erkennung etc. Mit diesem Merkmal kann unter Umständen die Anzahl von Desinfektionen verringert werden, wenn die Anwender den Touchscreensensor nur in jeweils unterschiedlichen Bereichen benutzen.

Gemäß einem Aspekt bezieht sich die Erfindung auch auf einen Touchscreen zur Steuerung eines medizinischen Gerätes, insbesondere eines Dialysegerätes, mit einem Touchscreensensor. Dabei umfasst der Touchscreen eine Desinfektionsüberwachungseinheit, wie vorstehend beschrieben.

Gemäß einer vorteilhaften Weiterbildung kann der Touchscreen ein Sicherheitsmodul umfassen, das dazu ausgebildet ist, den Touchscreen oder dessen Sensor automatisch anders zu konfigurieren, falls der Desinfektionsstatussensor einen "nicht desinfizierten" Desinfektionszustand erfasst hat. Darunter ist ein modifizierter Bildschirmaufbau zu verstehen, bei dem die Interaktions- und Schaltflächen und Eingabefelder, die also eine Berührung erfordern, in einem anderen Bildschirmsegment dargestellt werden und sozusagen an eine Position verschoben werden, die als desinfiziert erfasst worden ist. Zusätzlich kann ein entsprechender Warnhinweis und/oder ein Aufforderungssignal ausgegeben werden. Damit kann eine Bedienung des Touchscreensensors im nicht desinfizierten Zustand ausgeschlossen werden.

Gemäß einer anderen Weiterbildung kann der Touchscreen ein Markiermodul umfassen, das dazu ausgebildet ist, den erfassten Desinfektionszustand des Touchscreensensors oder die jeweiligen Desinfektionszustände der einzelnen Segmente des Touchscreensensors jeweils separat und ortsaufgelöst auf dem Touchscreensensor zu kennzeichnen. Damit erhält der Anwender vorteilhafterweise einen Hinweis über die verwendbaren Bereiche und Segmente des Displays, die er - da sie desinfiziert sind - noch bedienen kann.

Gemäß einer anderen vorteilhaften Weiterbildung umfasst der Touchscreen weiterhin einen Treiber zur Interaktion des Touchscreens mit dem medizinischen Gerät, der mit den vom Desinfektionsstatussensor ermittelten Daten zur desinfektionsstatusabhängigen Ansteuerung des Touchscreens betrieben und/oder angesteuert wird. Der Treiber kann mit dem Betriebssystem des medizinischen Gerätes interagieren und kann direkt auf dem medizinischen Gerät angeordnet sein oder auf einer separaten Einheit, die in Datenaustausch mit dem medizinischen Gerät steht.

In einem weiteren Aspekt bezieht sich die Erfindung auf ein medizinisches Gerät, insbesondere auf ein Dialysegerät, mit einem Touchscreen, wie er vorstehend beschrieben worden ist. Das Gerät kann auch eine Ausgabeeinheit umfassen oder an diese über eine Datenverbindung angeschlossen sein, wobei die Ausgabeeinheit dazu ausgebildet ist, den erfassten Desinfektionszustand auszugeben.

In einem weiteren Aspekt bezieht sich die Erfindung auf ein medizinisches System zur Steuerung eines medizinischen Gerätes, insbesondere eines Dialysegerätes, umfassend:
- Einen Touchscreen als Eingabe- und Ausgabeschnittstelle zur Steuerung des medizinischen Gerätes;
- Eine Desinfektionsüberwachungseinheit, die einen Desinfektionsstatussensor umfasst, der dazu ausgebildet ist, einen Desinfektionszustand des Touchscreens bzw. des Touchscreensensors zu erfassen.

In einer bevorzugten Ausführungsform der Erfindung umfasst das System weiterhin:
- Eine Ausgabeeinheit, die dazu ausgebildet ist, den erfassten Desinfektionszustand auszugeben. Dies kann durch Ausgabe eines akustischen und/oder optischen Signals erfolgen oder durch Weiterleitung des erfassten Desinfektionszustandes an eine andere datentechnisch gekoppelte Instanz, z.B. eine zentrale Überwachungseinheit.

Weiterhin kann das System ein Sicherheitsmodul umfassen, das dazu bestimmt ist, automatisch die Modifikation des Bildschirmaufbaus des Dialysegerätes oder des Touchscreens zu triggern (Darstellen der Interkations- und Schaltfläche ausschließlich in als desinfiziert erfassten Segmenten).

In einem weiteren Aspekt bezieht sich die Erfindung auf ein Verfahren zur Steuerung eines medizinischen Gerätes, insbesondere eines Dialysegerätes, über einen Touchscreen, umfassend folgende Verfahrensschritte:
- Automatisches oder manuelles Erfassen eines Desinfektionszustandes auf dem Touchscreen bzw. auf dem Touchscreensensor;
- Bereitstellen des erfassten Desinfektionszustandes
   indem Touchscreensensorkontakte auf dem Touchscreensensor ausgewertet werden und
   indem eine Fluidauswertung ein Vorhandensein von Desinfektionsmittel, in der Regel von Desinfektionsflüssigkeit, überprüft,
   wobei die Fluidauswertung das Vorhandensein von Desinfektionsmittel nur während einer Desinfektionsphase und nicht während der Gerätesteuerung überprüft,
   wobei flächige Bewegungsmuster auf dem Touchscreensensor zur Desinfektion automatisch erkannt und von Punkt- oder Druckbedienungen zur Eingabe von Daten bzw. zur Gerätesteuerung differenziert werden, und/oder
- wobei Bedienungen oder Kontakte des Touchscreensensors mittels eines optischen Sensors erfasst und an die Desinfektionsüberwachungseinheit zur Auswertung weitergeleitet werden.

Gemäß einer anderen vorteilhaften Weiterbildung ist die Erfassung des Desinfektionszustandes in einen Regelkreis eingebunden, um den Touchscreen und die Darstellung der Elemente auf dem Touchscreen zu regeln. Dabei soll ein modifizierter Bildschirmaufbau ausgelöst werden, sobald zumindest ein Segment des Touchscreensensors als nicht desinfiziert erfasst worden ist. Bei dem modifizierten Bildschirmaufbauprozess und der entsprechenden Ansteuerung werden alle Interaktionsflächen, touch keys, Bedienfelder und/oder sonstigen Schaltelemente, die einen Kontakt mit dem Anwender erfordern, ausschließlich in einem Bereich des Touchscreens dargestellt, der als desinfiziert erfasst wurde. Natürlich können auch mehrere einzelne separate Segmente oder Bereiche des Touchscreens bzw. des Touchscreensensors als desinfiziert identifiziert werden. In diesem Fall dienen all diese Bereiche als Grundlage für die Anzeige.

Gemäß einer anderen vorteilhaften Weiterbildung erfolgt das Erfassen des Desinfektionszustandes nur in bestimmten bzw. konfigurierbaren Fällen. Dazu kann ein Überwachungssignal auf dem Touchscreen, dem medizinischen Gerät oder auf einer separaten, datentechnisch gekoppelten Einheit abgegriffen werden, die den Touchscreen einer Desinfektionsüberwachung aussetzt. So kann der Anwender z.B. die gezielte Überprüfung der Desinfektion anstoßen, indem er das Überwachungssignal eingibt. In einer weiteren Ausführungsform der Erfindung ist es vorgesehen, dass das Überwachungssignal immer dann automatisch generiert wird, wenn ein Nutzerwechsel (die Erkennung eines Nutzerwechsels wird weiter unten näher erläutert) erkannt worden ist. Ein Nutzerwechsel führt somit automatisch zu einer Überprüfung der Desinfektion. Auch kann ein Zeitraum eingestellt werden, zu dem die Überwachungen (z.B. in einem festen Turnus) durchgeführt werden sollen. Weiterhin kann auch ein vordefinierbarer Zeitabstand zwischen zwei Bedienungen des Touchscreensensors zur automatischen Auslösung der Überwachung des Desinfektionszustandes führen.

Gemäß einer anderen vorteilhaften Weiterbildung wird der Desinfektionszustand automatisch erfasst. Dies erfolgt, indem Touchscreensensorkontakte (wie Eingaben, Bedienungen, Wischbewegungen, Drückbedienungen etc.) auf dem Touchscreensensor einer Auswertelogikeinheit zugeführt werden, die zur Auswertung der Kontakte bestimmt ist. Üblicherweise ist die Auswertung auf einen vordefinierbaren Überwachungszeitraum beschränkt, der der aktuellen Gerätesteuerung zeitlich vorausgeht. Dies ist üblicherweise voreingestellt. Es ist jedoch auch möglich, das Zeitfenster und die Dauer des Überwachungszeitraumes gezielt zu spezifizieren. Damit kann den klinischen Anforderungen vor Ort Rechnung getragen werden. Wenn z.B. ohnehin eine Desinfektion des Gerätes in einem Tagesrhythmus vorgesehen ist, dann kann der Überwachungszeitraum kürzer als ein Tag sein. Dies spart Ressourcen hinsichtlich der Erfassung, Verarbeitung und Speicherung der Daten. Zur Erfassung des Desinfektionszustandes werden Touchscreensensorkontakte, die im vordefinierten, zurückliegenden Zeitraum erfasst wurden, ausgewertet. Dies kann durch einen Sensor im Touchscreen selbst ausgeführt werden. Dies kann auch durch ein Softwaremodul ausgeführt werden, das zur Auswertung der Touchscreensensorkontakte bestimmt ist und zwischen flächigen Wischbewegungen und punktuellen Druckkontakten differenziert. Alternativ kann ein optischer Sensor, z.B. eine Kamera, zur Erfassung der Kontakte vorgesehen sein. Alternativ oder kumulativ kann der Anwender nach einer Desinfektion auch manuell ein Schaltelement zum Erfassen des Desinfektionszustandes betätigen.

Gemäß einer anderen vorteilhaften Weiterbildung wird automatisch ein Aufforderungssignal ausgegeben, falls als Desinfektionszustand "nicht desinfiziert" für zumindest ein Segment des Touchscreensensors erfasst worden ist. Dies kann auch der Fall sein, wenn zwar der vollständige Touchscreensensor desinfiziert worden ist, aber nicht in ausreichender Qualität. Für die Beurteilung der Qualität kann die desinfizierte Fläche und/oder die Dauer der Desinfektion berücksichtigt werden.

Eine weitere Aufgabenlösung besteht in einem Computerprogrammprodukt, das in einen Speicher eines Computers oder eines elektronischen oder medizintechnischen Gerätes geladen oder ladbar ist mit einem Computerprogramm zur Durchführung des oben näher beschriebenen Verfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1: zeigt in einer schematischen Darstellung ein Dialysegerät mit einem Touchscreen, der gemäß einer Ausführungsform der Erfindung mit einer Desinfektionsüberwachungseinheit ausgebildet ist.
- Fig. 2: ist eine weitere schematische Darstellung eines Dialysegerätes mit einem Touchscreen und weiteren Bauteilen.
- Fig. 3: zeigt ein weiteres Ausführungsbeispiel eines medizinischen Systems zur Steuerung eines Dialysegerätes.
- Fig. 4: ist eine exemplarische Schema-Ansicht eines Touchscreens mit unterschiedlich markierten Segmenten.
- Fig. 5: ist eine weitere exemplarische Prinzip-Ansicht eines Touchscreens mit Ein- und Ausgabefeldern zur Steuerung des Dialysegerätes.
- Fig. 6: zeigt eine schematische Darstellung unterschiedlicher Prozesse im Rahmen der Desinfektionsüberwachung über die Zeit.
- Fig. 7: ist eine beispielhafte schematische Darstellung für einen modifizierten Bildschirmaufbau.

### Detaillierte Beschreibung der Figuren

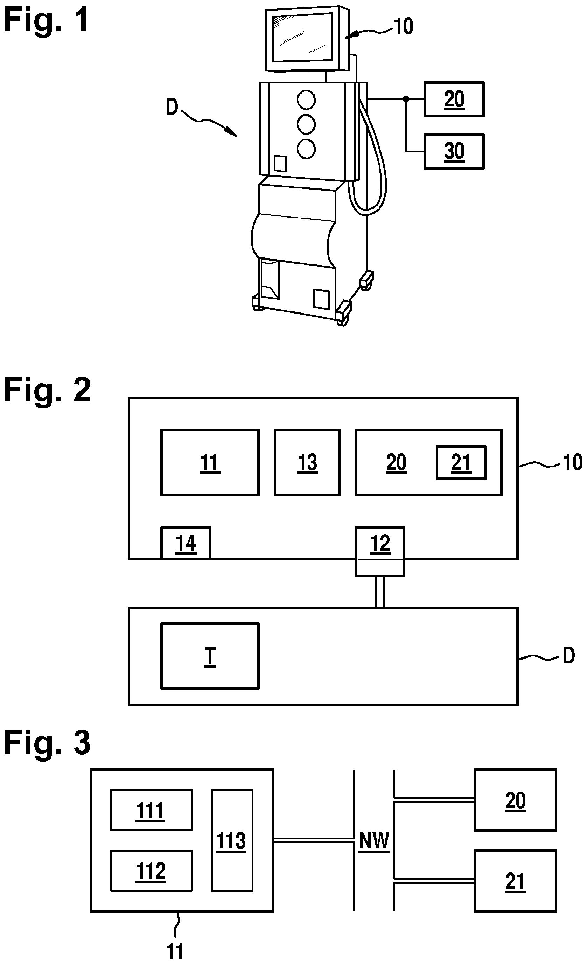

Im Folgenden wird die Erfindung anhand von Ausführungsbeispielen im Zusammenhang mit den Figuren näher beschrieben. Die Erfindung betrifft die Überwachung einer Desinfektion eines Touchscreens 10, der zur Steuerung eines medizinischen Gerätes D, insbesondere eines Dialysegerätes verwendet wird. **Figur 1** zeigt schematisch ein Dialysegerät D mit einem Touchscreen 10, einer Desinfektionsüberwachungseinheit 20 und einer Ausgabeeinheit 30, deren Funktionsweise später im Detail erläutert wird. Der Touchscreen 10 (der im Folgenden auch als Monitor bezeichnet wird) umfasst einen Touchscreensensor 11 zur Erfassung von Eingabesignalen und ein Display als Ausgabeschnittstelle, die üblicherweise übereinander angeordnet sind. Der auf einen Desinfektionszustand zu überprüfende Bereich des Touchscreensensors 11 stimmt somit üblicherweise mit dem Bereich des Touchscreens 10 überein.

Bei der Steuerung eines Dialysegerätes wird der Touchscreensensor 11 häufig von mehreren Personen berührt. Dabei besteht die Gefahr von Kreuzkontaminationen und der Übertragung von Keimen. Um dies zu vermeiden, wird der Touchscreensensor 11 regelmäßig desinfiziert und danach wieder bedient. Die Erfindung stellt nun darauf ab, den Zustand (desinfiziert oder nicht bzw. nicht ausreichend desinfiziert) des Touchscreensensors 11 und auch einzelnen Segmenten S des Touchscreensensors 11 zu erfassen und anzuzeigen, damit der Anwender weiß, ob der Monitor ohne Gefahr bedient werden kann oder ob zunächst eine Desinfektion durchgeführt werden muss. Dabei dient ein Desinfektionsstatussensor 21 zur Erfassung des Desinfektionszustandes des Touchscreensensors 11.

Der Touchscreensensor 11 ist Bestandteil eines Touchscreens bzw. Touchscreensystems 10. Der Touchscreen 10 umfasst neben dem Display und dem Touchscreensensor 11 auch üblicherweise einen Controller 12 und einen Treiber T. Der Touchscreensensor 11 wird zur Eingabe von Daten in Rahmen der Bedienung und Steuerung des Gerätes D verwendet. Der Controller 12 dient zur Steuerung des Touchscreensensors 11 und verbindet die Maschine bzw. das jeweilige medizinische Gerät D in datentechnischer Hinsicht mit dem Touchscreensensor 11. Der Controller 12 kann in Form einer PC-Karte oder eines elektronischen Moduls ausgebildet sein. Der Treiber T ist üblicherweise ein Software Treiber und kann mit einem Betriebssystem des medizinischen Gerätes D interagieren. Es definiert, wie die vom Touchscreensensor 11 erfassten Eingabesignale interpretiert werden sollen und definiert, wie die vom medizinischen Gerät D ausgegebenen Signale auf dem Touchscreensensor 11 dargestellt werden sollen.

Der Touchscreen 10 kann in unterschiedlichen Varianten nach demselben Grundmuster aufgebaut sein. Er umfasst eine berührungsempfindliche Oberfläche - den Touchscreensensor 11 - und den Controller 12, der die Signale an der Oberfläche misst und an ein Betriebssystem weitergibt. Das Betriebssystem und der Controller 12 übersetzen dann die Fingerbewegungen bzw. Kontakte auf dem Touchscreensensor 11 in Signale, die zur Steuerung des Dialysegerätes D verwendet werden. Die Sensorik, welche die Signale erzeugt, kann dabei auf verschiedenen physikalischen Prinzipien beruhen. Vorzugsweise wird für die medizinischen Geräte D eine kapazitive Sensortechnik mit Muli-Touch Funktionalität verwendet. Kapazitive Touchsreensensoren 11 sind aus einem zweischichtigen Koordinatennetz aus Elektroden aufgebaut, die sich in einer Schicht als Spalten und in der anderen als Zeilen anordnen. Zwischen den Elektroden befindet sich ein isolierendes Material als Dielektrikum. An der unteren Seite ist ein Schaltkreis angebracht, der ständig die Kapazität an den Kreuzungspunkten der Elektroden misst. An der Oberseite sorgt eine isolierende Schutzschicht, üblicherweise aus Glas, dafür, dass die Elektroden nicht beschädigt werden und der Finger gut über die Oberfläche gleiten kann. Da ein Finger elektrisch leitend ist, können Ladungen an ihm abfließen, sobald er die Oberfläche des Touchscreensensors 11 berührt, was zu einer messbaren Änderung der Kapazität an den jeweiligen Elektrodenschnittpunkten führt. Die Veränderung der Kapazität wird von einem Mikrokontroller oder einem Touchscreenprozessor erfasst und verarbeitet, um Berührungsdaten zu erzeugen, die an ein Betriebssystem (insbesondere des medizinischen Gerätes D) weitergeleitet werden können.

**Figur 2** beschreibt schematisch weitere Bauteile des medizinischen Gerätes D und des Touchscreens 10. Der Treiber T kann in dem Gerät D angeordnet sein, während der Controller 12, der als Microcontroller ausgebildet sein kann, direkt in dem Touchscreen 10 angeordnet ist oder die Verbindung dazu darstellt und sozusagen als Schnittstelle ausgebildet ist. Alternativ kann der Controller 12 auch als separate Instanz ausgebildet sein, die über eine Datenverbindung angeschlossen ist.

Der Touchscreen 10 umfasst als wesentlichen Bestandteil den Touchscreensensor 11 zur Detektion von Berührungen auf den Eingabefeldern zur Steuerung des Gerätes D, die im Zusammenhang mit den Figuren 3 bis 5 näher beschrieben werden. Erfindungsgemäß umfasst der in Figur 2 dargestellte Touchscreen 10 des Weiteren eine Desinfektionsüberwachungseinheit 20, die wiederum den Desinfektionsstatussensor 21 zur automatischen Erfassung des Desinfektionszustandes umfassen kann.

Gemäß einer vorteilhaften Ausführungsform der Erfindung umfasst er auch ein Markiermodul 13, das dazu ausgebildet ist, alle als desinfiziert erfasste Segmente S1 des Touchscreens 10 bzw. des Touchscreensensors 11 und/oder als nicht-desinfizierte Segmente S2 anzuzeigen.

Vorzugsweise erfolgt diese Anzeige unmittelbar auf dem Touchscreen 10 und/oder auf einer Ausgabeeinheit 30. Vorzugsweise ist die Ausgabeeinheit 30 identisch mit dem Touchscreen 10 oder auf diesem ausgebildet. Dies hat den Vorteil, dass der Nutzer direkt erkennen kann, welche Segmente S des Touchscreensensors 11 als desinfiziert erfasst wurden und er somit unmittelbar bedienen kann. Dazu kann ein spezifisches Symbol auf dem Touchscreen ausgegeben werden, z.B. in Form einer Flagge, die den Zustand repräsentiert (grüne Flagge steht für "desinfiziert" und rote Flagge für "nicht-desinfiziert").

In einer einfacheren Variante der Erfindung kann die Ausgabeeinheit 30 auch ein separates Bauteil, z.B. eine LED sein, die grün leuchtet, wenn alle Segmente S des Touchscreensensors 11 desinfiziert sind und die andernfalls rot leuchtet.

Gemäß einer weiteren Ausführungsform der Erfindung umfasst der Touchscreen 10 ein Sicherheitsmodul 14. Das Sicherheitsmodul 14 ist dazu ausgebildet ist, den Touchscreen 10 automatisch modifiziert anzusteuern und einen neuen Bildschirmaufbau zu triggern, falls ein Desinfektionsstatussensor 21 einen "nicht desinfizierten" Desinfektionszustand erfasst hat. Dies hat den Zweck Kreuzkontaminationen sicher zu vermeiden, indem alle Eingabe-, Schalt- und Interaktionsflächen 111, 112 in als desinfiziert erfassten Segmenten S dargestellt werden.

In einer einfacheren Ausführungsform der Erfindung umfasst die Desinfektionsüberwachungseinheit 20 keinen Desinfektionsstatussensor 21, wie in Figur 2 dargestellt, sondern lediglich ein Schaltelement 15 (das in Figur 2 nicht dargestellt ist). Das Schaltelement 15 kann als Schaltfläche (z.B. als touch key pad) auf dem Touchscreen 10 oder als mechanischer Schalter am Gerät D und/oder am Touchscreen 10 ausgebildet sein. Das Schaltelement 15 dient dazu, einen Desinfektionsstatus seitens des Anwenders einzugeben, falls der Desinfektionsstatus nicht automatisch sondern manuell erfasst werden soll. Nach einer ausgeführten Desinfektion kann das Personal, den Schalter 15 betätigen, um dem System zu signalisieren, dass der Touchscreensensor 11 desinfiziert worden ist. Vorzugweise erfolgt die Erfassung des Desinfektionsprozesses jedoch automatisch. Dazu können bisherige Bedienkontakte auf dem Touchscreensensor 11 ausgewertet werden.

In **Figur 3** ist eine alternative Ausführungsform der Erfindung gezeigt. Der Touchscreensensor 11 dient dazu, Berührungen auf den Schalt- und Interaktionsflächen 111, Eingabefeldern 112 zu detektieren. Die Ausgabeschnittstelle bzw. das Display des Touchscreens 10 befindet sich im selben Bereich und dient zur Anzeige der Ausgabefelder 113. Die Anzeige bzw. Darstellung kann z.B. mittels des Treibers T und/oder des Controllers 12 gesteuert werden. Der Touchscreensensor 11 steht über ein Bussystem oder Netzwerk NW mit der Desinfektionsüberwachungseinheit 20 und mit dem Desinfektionsstatussensor 21 in Datenaustausch. In diesem Ausführungsbeispiel ist der Desinfektionsstatussensor 21 als separates Modul vorgesehen. Dabei kann es sich z.B. um eine zuschaltbare Kamera oder um einen sonstigen Sensor handeln, der zur Erfassung der Kontakte auf dem Touchscreensensor 11 bestimmt ist. Die erfassten Signale werden der Desinfektionsüberwachungseinheit 20 zur Auswertung zugeführt. Durch bekannte Methoden der Mustererkennung kann identifiziert werden, ob der Touchscreensensor 11 mit einem Tuch oder Lappen desinfiziert worden ist. Alternativ zur optischen Erfassung kann der Desinfektionsstatussensor 21 als Feuchtigkeitserfassungssensor ausgebildet sein, der das Vorhandensein von Flüssigkeit bzw. Feuchtigkeit des Desinfektionsmittels erkennt. Der Desinfektionsstatussensor 21 kann auch als Modul in dem Treiber T und/oder in dem Controller 12 und insbesondere dazu ausgebildet sein, die Kontakte auf dem Touchscreensensor 11 auszuwerten. Insbesondere wird das Bewegungsmuster ausgewertet, nämlich dahingehend, ob es sich um dedizierte Bedienungen der Schalt- und Interaktionsflächen 111 bzw. um Eingaben in die Eingabefelder 112 zur Steuerung des Gerätes D handelt oder um flächige Wischbewegungen zum Zwecke der Desinfektion. Im letzteren Fall kann zusätzlich der Bereich der Wischbewegungen (auf dem Touchscreensensor 11) und die Zeitdauer der Wischbewegungen für die Desinfektion und den Zeitpunkt (z.B. Beginn und Ende) erfasst werden. Diese Daten werden als desinfektionsrelevante Meta-Daten in einem Speicher gespeichert und können für spätere Abfragen verwendet werden.

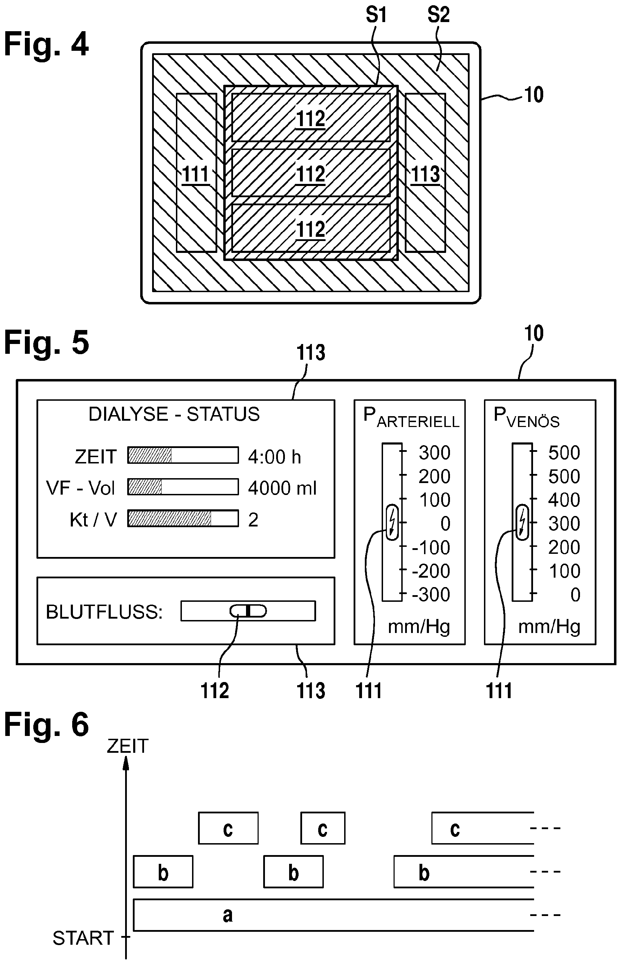

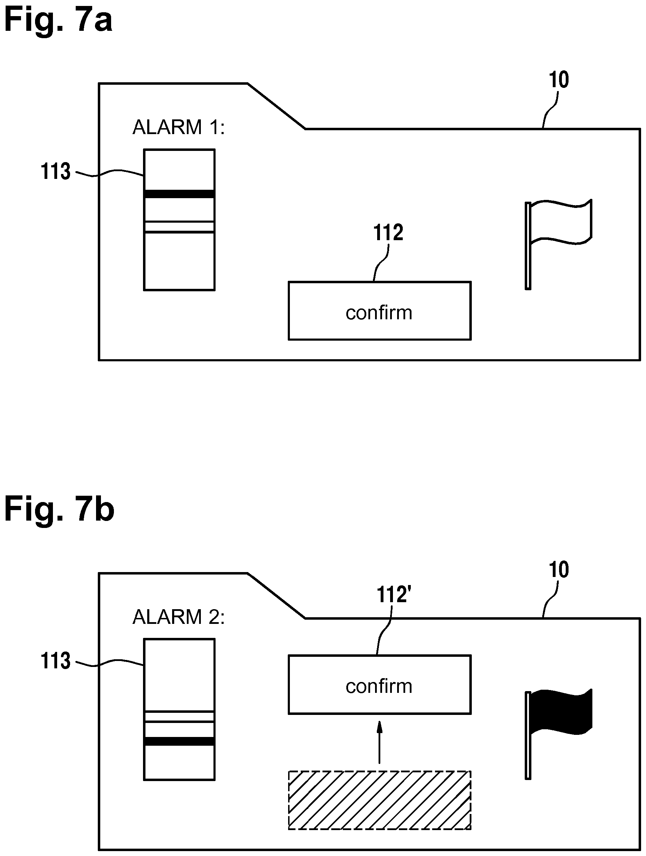

Die erfassten Desinfektionsdaten und Meta-Daten können insbesondere zur Markierung von Bildschirmflächen verwendet werden, wie dies näher in Zusammenhang mit Fig. 4 erläutert wird. Auf dem dargestellten Touchscreen 10 sind beispielhaft wieder Schalt- und Interaktionsflächen 111, Eingabefelder 112 dargestellt, die zur Steuerung des Dialysegerätes D kontaktiert werden müssen. Auf dem Touchscreen 10 werden zudem die Ausgabefelder 113 dargestellt. Nach Erfassung des Desinfektionsstatus können Bereiche auf dem Screen markiert werden, die als desinfizierte Segmente S1 erfasst wurden und/oder andere Bereiche, die als nicht-desinfizierte Segmente S2 erfasst wurden. In dem in Figur 4 gezeigten Beispiel konnte nur der innere Bereich des Sensors ausreichend desinfiziert werden, während die äußeren Randbereiche nicht desinfiziert sind (Segment S2). Damit liegen nur die Eingabefelder 112 vollständig im desinfizierten Bereich. Es wäre also in diesem Fall ohne die Gefahr von Kreuzkontaminationen hervorzurufen möglich, die mittleren Eingabefelder 112 weiter zu bedienen. Die in Figur 4 beispielhaft dargestellte weitere Interaktionsfläche 111 darf nicht ohne vorherige - gegebenenfalls nochmalige - Desinfektion berührt werden. Falls die Desinfektionsüberwachungseinheit 20 nicht desinfizierte oder nicht ausreichend oder vollständig desinfizierte Bereiche erkannt hat, kann automatisch ein Aufforderungssignal generiert werden, um eine Desinfektion zu triggern. Vorzugsweise wird in dem in Figur 4 gezeigten Beispiel ein modifizierter Bildschirmaufbau ausgelöst, der eingehender in Zusammenhang mit Figur 7 weiter unten beschrieben werden wird. Dabei ist es vorgesehen, das Feld 111 an eine als desinfiziert erkannte Position zu verschieben. Das kann erfolgen, indem die Schaltflächen 112 verkleinert werden, um zusätzlich das Feld 111 im Bereich S1 darstellen zu können. Falls dies nicht möglich ist, kann eine vollständige Verschiebung des Fensters ausgelöst werden, derart, dass alle Eingabe- und Schaltflächen 111, 112 im desinfizierten Segment S1 dargestellt werden. Falls auch dies nicht möglich sein sollte, wird ausschließlich das Warnsignal ausgegeben. Aufgrund des hohen Sicherheitsrisikos können hier unterschiedliche Ausgabeformate für das Warnsignal kombiniert werden (akustische und visuelle Ausgabe und zusätzlich ein Warnsignal).

**Figur 5** zeigt ein weiteres Beispiel für einen Touchscreen mit einem Touchscreensensor 11, der zur Erfassung von Kontakten auf Eingabefeldern 112 und Interaktionsflächen 111 bestimmt ist. Der Touchscreen 10 dient zur Darstellung aller Felder und insbesondere auch von Ausgabefeldern 113. Da das Feld 113 nur ein Ausgabefeld ist, wäre es in diesem Beispiel zunächst ohne Folge, wenn nur dieses Segment im Bereich des Feldes 113 als nicht desinfiziert erkannt worden ist, da hier keine Daten eingegeben und somit kein Anwenderkontakt wahrscheinlich ist. In einer Variante kann es jedoch aus Sicherheitsgründen voreingestellt sein, dass immer ein Aufforderungssignal ausgegeben wird, falls zumindest ein Segment S des Touchscreensensors 11 als nicht desinfiziert erfasst worden ist. In dem in Figur 5 gezeigten Beispiel sind das Feld 112 als Schieberegler zum Blutfluss und die Felder 111 ebenfalls als Schieberegler für den arteriellen und venösen Blutdruck und damit jeweils als Eingabefeld ausgebildet. Diese Felder müssen in einem desinfizierten Segment dargestellt werden.

**Figur 6** stellt unterschiedliche Verfahrensschritte dar. Nach dem Start und dem Hochfahren und Aufrüsten des Gerätes D kann die Desinfektionsüberwachung aktiviert werden. In einer Variante der Erfindung kann diese während des Betriebs des Gerätes D aktiviert und deaktiviert werden, um unnötige Überprüfungen der Desinfektion zu vermeiden.

Bei dem Bezugszeichen a erfolgt die Inbetriebnahme des Touchscreens 10 zur Steuerung des Gerätes D. Daraufhin kann zu vorkonfigurierbaren Ereignissen in Schritt b das Erfassen von Kontakten oder Berührungen auf dem Touchscreensensors 11 und das Speichern der erfassten Daten ausgeführt werden. Die vorkonfigurierbaren Ereignisse können zeitdefiniert sein (z.B. alle 10 Minuten) oder durch ein Bestätigungssignal ausgelöst werden oder durch Eintreten eines vordefinierten Ereignisses ausgelöst werden (z.B. Erfassen einer Bildschirmbewegung, Erfassung eines Nutzerwechsels, etc.). Üblicherweise ist es voreingestellt, dass der Desinfektionszustand und damit insbesondere die Kontakte auf dem Touchscreensensor 11 während des Betriebs und der Steuerung des Dialysegerätes D erfasst werden, um eine vollständige Datenbasis zu haben. Es kann auch voreingestellt werden, dass das Erfassen des Desinfektionszustandes nicht nur während des Betriebs, sondern im Rahmen des Betriebs erfolgt, und somit beispielsweise auch während einer Wartungsphase ausgeführt werden kann. Ebenso kann voreingestellt sein, dass in Ausnahmefällen, z.B. während Wartungsphasen, eine Erfassung ausgeschlossen wird. Wie in Figur 6 ersichtlich, muss das Erfassen der Kontakte also nicht zwangsläufig kontinuierlich erfolgen. Daraufhin (nach dem Erfassen) kann in Schritt c das Auswerten der erfassten Kontaktdaten und das Bereitstellen eines Auswerteergebnisses, z.B. in Form von markierten Monitorsegmenten S1, S2, ausgeführt werden. Das Erfassen der Kontaktdaten und deren Auswertung sind grundsätzlich in Hinblick auf den Zeitraum und den Ort (den jeweiligen funktionalen Baustein) der Verarbeitung unabhängig. Die Auswertung kann beispielsweise aus Gründen der Ressourcenbeschränkung ebenfalls ereignisbasiert getriggert werden, wie oben im Zusammenhang mit der Datenerfassung bereits beschrieben. So kann die Auswertung z.B. periodisch oder ereignisbasiert sporadisch ausgeführt werden.

**Figur 7** zeigt an einem Beispiel einen modifizierten Bildschirmaufbau. In **Figur 7a** ist auf der linken Seite auf schematische Weise ein Ausgabefeld 113 für einen Alarm 1 dargestellt. Der weiße Querbalken soll einen Grenzwert kennzeichnen, der in dem in Figur 7a dargestellten Beispiel überschritten wird; dies soll durch den schwarzen Querbalken gekennzeichnet sein. Die Kenntnisnahme des Alarmsignals 1 soll durch den Anwender durch Betätigen eines Bestätigungsfeldes 112 ("confirmation") bestätigt bzw. quittiert werden. Dazu muss der Anwender das Eingabefeld 112, das als Button oder Knopf ausgebildet sein kann, berühren. Das erfindungsgemäße Verfahren überprüft insbesondere vor einem Bedienkontakt auf dem Touchscreensensor 11 dessen Desinfektionsstatus. In diesem Fall ist der Bereich als desinfiziert erfasst und somit kann das Bestätigungsfeld 112 an seiner ursprünglichen Position auf dem Touchscreen 10 verbleiben und so dargestellt werden. Gemäß einer bevorzugten Ausführungsform der Erfindung kann der erfasste Desinfektionsstatus in einem Symbol auf der Oberfläche repräsentiert sein. Dies ist in Fig. 7a als weiße Flagge dargestellt, die symbolisieren soll, dass das confirm-Feld 112 ohne Bedenken bedient werden kann, da die weiße Flagge einen desinfizierten Zustand kennzeichnet.

Im Fall von **Figur 7b** soll nur ein Alarmsignal 2 bestätigt werden, das in dem dargestellten Beispiel seinen Grenzwert unterschreitet (der schwarze Balken, der den IST-Wert kennzeichnet liegt unter dem Grenzwert, der als Referenz bzw. als SOLL-Wert dient). In diesem Fall ist allerdings das Bildschirmsegment S, in dem das Bestätigungsfeld ursprünglich dargestellt werden sollte als nicht-desinfiziert erfasst. Die Desinfektionsüberwachungseinheit 20 und insbesondere das Sicherheitsmodul 14 werden nun automatisch aktiviert und triggern eine Ausgabe des Zustandes über das Symbol der schwarzen Flagge, die den nicht-desinfizierten Zustand repräsentiert (und möglicherweise ein zusätzliches Warnsignal) und eine modifizierte Bildschirmansteuerung. Bei der modifizierten Bildschirmansteuerung werden die Positionen der darzustellenden Eingabefelder 112 mit denjenigen Segmenten S des Touchscreensensors 11 abgeglichen, die als desinfiziert erfasst worden sind. Das Eingabefeld 112' wird somit an eine andere Position auf dem Touchscreen 10 verschoben und kann ohne die Gefahr von Kreuzkontaminationen bedient werden. In Figur 7b sind somit zwei unterschiedliche Zeitphasen dargestellt. In Figur 7b bezieht sich die Darstellung des gestrichelt und gepunktet umrandet dargestellten Feldes 112 an seiner ursprünglich geplanten, aber nicht-desinfizierten Position auf eine erste Zeitphase, in der auch die Darstellung der schwarzen Flagge erfolgt, um zu kennzeichnen, dass dieser Bereich nicht-desinfiziert ist. In einer zeitlich nachgelagerten Zeitphase wird dann der Bildschirm bzw. der Touchscreen 10 nochmals neu aufgebaut, so dass das confirm-Feld nun an eine neue, desinfizierte Position 112` (dargestellt mit durchgezogener Linie, nicht schraffiert) verschoben ist. Bei erfolgter Desinfektion erscheint die schwarz Flagge nicht mehr. Die schwarze Flagge erscheint jedoch bei nicht erfolgter Desinfektion dann weiterhin für den nicht-desinfizierten Bereich.

Alternativ können nicht nur einzelne Felder 111, 112 verschoben werden, sondern die jeweilige Gesamtmaske oder das jeweilige Fenster wird derart an eine neue Position auf dem Touchscreen 10 verschoben, dass alle Eingabe- und Interaktionsflächen 111, 112 des Fensters sich in einem als desinfiziert erfassten Segment S befinden. Dies hat den Vorteil, dass der Anwender seine gewohnte Oberfläche mit den üblichen Anordnungen der jeweiligen Elemente 111, 112, 113 in relativ zueinander unveränderter Position (nur an einer anderen Gesamtposition des Fensters auf dem Touchscreen 10) benutzen kann.

In anderen Ausführungsform der Erfindung kann das Symbol zur Repräsentation des Desinfektionszustandes möglichst immer an derselben Position auf dem Monitor dargestellt werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung neben dem Touchscreen andere Bedien- oder Steuerelemente des medizinischen Gerätes auf deren Desinfektionszustand zu überprüfen. Dabei kann es sich z.B. um handbetätigte Bedienelemente, Joysticks, eine Maus, eine herkömmliche Tastatur oder dergleichen handeln. Für einen Fachmann ist es insbesondere offensichtlich, dass die Erfindung nicht nur für Dialysegeräte angewendet werden kann, sondern auch für andere medizinische Geräte D, die über Bedienelemente, wie Touchscreensensoren 11 oder andere Elemente, bedient und gesteuert werden müssen und die vor einer Kontamination geschützt werden sollen.

Des Weiteren können die Bauteile des medizinischen Systems zur Überwachung des Desinfektionszustandes auf mehrere physikalische Produkte verteilt realisiert werden kann.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHEN

- D: Medizinisches Gerät, insbesondere Dialysegerät
- 10: Touchscreen
- 11: Touchscreensensor
- 12: Controller
- 13: Markiermodul
- 14: Sicherheitsmodul
- 15: Schaltelement
- 111: Schalt- und Interaktionsfläche
- 112: Eingabefeld
- 113: Ausgabefeld
- S: Segment des Touchscreensensors
- S1, S2,..: erstes, zweites Segment des Touchscreensensors
- 20: Desinfektionsüberwachungseinheit
- 30: Ausgabeeinheit
- 21: Desinfektionsstatussensor
- T: Treiber
- NW: Netzwerk

- a: Betätigen und Steuern des medizinischen Gerätes
- b: Erfassen der Kontakte auf dem Touchscreensensor 11
- c: Auswerten der erfassten Kontakte, ob sie einer Desinfektion zugeordnet werden können oder nicht und Bereitstellen eines Auswerteergebnisses in Form eines Desinfektionszustandes

## Patentansprüche

1. Desinfektionsüberwachungseinheit (20) für einen Touchscreensensor (11) zur Steuerung eines medizinischen Gerätes (D), insbesondere eines Dialysegerätes, wobei die Desinfektionsüberwachungseinheit (20) einen Desinfektionsstatussensor (21) umfasst, der dazu ausgebildet ist, den Desinfektionszustand des Touchscreensensors (11) automatisch zu erfassen,
wobei der Desinfektionsstatussensor (21) zumindest als Kamera und als Fluidauswerteeinheit ausgebildet ist,
wobei die Fluidauswerteeinheit dazu ausgebildet ist, ein Vorhandensein von Desinfektionsmittel, in der Regel von Desinfektionsflüssigkeit, zu überprüfen,
wobei die Fluidauswerteeinheit nur während einer Desinfektionsphase und nicht während der Gerätesteuerung gesteuert aktiviert wird,
wobei der Touchscreensensor (11) dazu ausgebildet ist, flächige Bewegungsmuster zur Desinfektion automatisch zu erkennen und von Punkt- oder Druckbedienungen zur Eingabe von Daten bzw. zur Gerätesteuerung zu differenzieren, und/oder wobei die Kamera dazu ausgebildet ist, Bedienungen oder Kontakte des Touchscreensensors (11) zu erfassen und an die Desinfektionsüberwachungseinheit (20) zur Auswertung weiterzuleiten.

2. Desinfektionsüberwachungseinheit (20) nach dem vorgehenden Patentanspruch, **dadurch gekennzeichnet, dass** der erfasste Desinfektionszustand als Zustandsvektor repräsentiert ist und kennzeichnet, ob eine Desinfektion stattgefunden hat und/oder dass der Zustandsvektor eine Bereichsangabe umfasst, die den Umfang der Desinfektion kennzeichnet und/oder weitere Meta-Daten zur Desinfektion umfasst.

3. Desinfektionsüberwachungseinheit (20) nach einem der vorhergehenden Patentansprüche, **dadurch gekennzeichnet, dass** die Desinfektionsüberwachungseinheit (20) eine Ausgabeeinheit (30) umfasst, die dazu ausgebildet ist, den erfassten Desinfektionszustand als Desinfektionssignal oder Desinfektionsnachricht auszugeben.

4. Desinfektionsüberwachungseinheit (20) nach einem der vorhergehenden Patentansprüche, **dadurch gekennzeichnet, dass** die Desinfektionsüberwachungseinheit (20) dazu ausgebildet ist, den Desinfektionszustand über eine Identifikation von zeitlich vorhergehenden Bewegungsmustern auf dem Touchscreensensor (11) zu erfassen.

5. Desinfektionsüberwachungseinheit (20) nach einem der vorhergehenden Patentansprüche, **dadurch gekennzeichnet, dass** die Desinfektionsüberwachungseinheit (20) ausgebildet ist, den Desinfektionszustand von mehreren vordefinierbaren Segmenten (S) des Touchscreensensors (11) zu erfassen.

6. Touchscreen (10) zur Steuerung eines medizinischen Gerätes (D), insbesondere eines Dialysegerätes, mit einem Touchscreensensor (11), **dadurch gekennzeichnet, dass** der Touchscreen (10) eine Desinfektionsüberwachungseinheit (20) nach einem der vorstehenden, auf die Desinfektionsüberwachungseinheit (20) gerichteten Patentansprüche umfasst.

7. Touchscreen (10) nach dem vorstehenden, auf den Touchscreen (10) gerichteten Patentanspruch, **dadurch gekennzeichnet, dass** der Touchscreen (10) ein Sicherheitsmodul (14) umfasst, das dazu ausgebildet ist, den Touchscreen (10) automatisch modifiziert anzusteuern, falls der Desinfektionsstatussensor einen "nicht desinfizierten" Desinfektionszustand erfasst hat, indem alle Schalt- und Interaktionsflächen und Eingabefelder (111, 112) in einem als desinfiziert erfassten Segment (S) des Touchscreensensors (11) dargestellt werden.

8. Touchscreen (10) nach einem der vorstehenden, auf den Touchscreen (10) gerichteten Patentansprüche, **dadurch gekennzeichnet, dass** der Touchscreen (10) weiterhin ein Markiermodul (13) umfasst, das dazu ausgebildet ist, den erfassten Desinfektionszustand des Touchscreensensors (11) oder die jeweils erfassten Desinfektionszustände der einzelnen Segmente (S) des Touchscreensensors (11) ortsaufgelöst auf dem Touchscreen (10) zu visualisieren und insbesondere zu markieren.

9. Touchscreen (10) nach einem der vorstehenden, auf den Touchscreen (10) gerichteten Patentansprüche, **dadurch gekennzeichnet, dass** der Touchscreen (10) weiterhin einen Treiber (T) zur Interaktion des Touchscreens (10) mit dem medizinischen Gerät (D) umfasst, der mit den von einem Desinfektionsstatussensor (21) ermittelten Daten zur desinfektionsstatusabhängigen Ansteuerung des Touchscreens (10) angesteuert wird.

10. Medizinisches Gerät (D), insbesondere Dialysegerät, mit einem Touchscreen (10) nach zumindest einem der vorstehenden auf den Touchscreen (10) gerichteten Patentansprüche.

11. Medizinisches Gerät (D) nach dem unmittelbar vorhergehenden Geräteanspruch, wobei das medizinische Gerät (D) ein Sicherheitsmodul (14) umfasst, das dazu ausgebildet ist, den Touchscreen (10) automatisch modifiziert anzusteuern, falls der Desinfektionsstatussensor (21) einen "nicht desinfizierten" Desinfektionszustand erfasst hat, indem alle Schalt- und Interaktionsflächen und Eingabefelder (111, 112) in einem als desinfiziert erfassten Segment (S) des Touchscreensensors (11) dargestellt werden.

12. Verfahren zur Steuerung eines medizinischen Gerätes (D), insbesondere eines Dialysegerätes, über einen Touchscreensensor (11), wobei während der Steuerung und/oder des Betriebs des medizinischen Gerätes (D) mittels des Touchscreensensors (11) ein Desinfektionszustand auf dem Touchscreensensor (11) automatisch erfasst und als Desinfektionssignal und/oder Desinfektionsnachricht bereitgestellt wird,
indem Touchscreensensorkontakte auf dem Touchscreensensor (11) ausgewertet werden und
indem eine Fluidauswertung ein Vorhandensein von Desinfektionsmittel, in der Regel von Desinfektionsflüssigkeit, überprüft,
wobei die Fluidauswertung das Vorhandensein von Desinfektionsmittel nur während einer Desinfektionsphase und nicht während der Gerätesteuerung überprüft, wobei flächige Bewegungsmuster auf dem Touchscreensensor (11) zur Desinfektion automatisch erkannt und von Punkt- oder Druckbedienungen zur Eingabe von Daten bzw. zur Gerätesteuerung differenziert werden, und/oder
wobei Bedienungen oder Kontakte des Touchscreensensors (11) mittels eines optischen Sensors erfasst und an die Desinfektionsüberwachungseinheit (20) zur Auswertung weitergeleitet werden.

13. Verfahren nach dem vorstehenden Verfahrensanspruch, **dadurch gekennzeichnet, dass** das Verfahren folgenden Verfahrensschritt umfasst:
- Steuern eines Bildschirmaufbaus auf Basis des erfassten Desinfektionszustandes, indem alle Eingabefelder (112) und Schalt- und Interaktionsflächen (112) ausschließlich in einem Bereich des Touchscreensensors (11) dargestellt werden, der als desinfiziert erfasst worden ist.

14. Verfahren nach einem der vorstehenden Verfahrensansprüche, **dadurch gekennzeichnet, dass** das Auswerten der Touchscreensensorkontakte auf dem Touchscreensensor (11) erfolgt, indem flächige Bewegungen identifiziert werden, insbesondere durch einen optischen Sensor.

15. Verfahren nach einem der vorstehenden Verfahrensansprüche, **dadurch gekennzeichnet, dass** das Erfassen des Desinfektionszustandes durch Erfassen eines Bestätigungssignals hin aktivierbar und deaktivierbar ist oder automatisch nach Eintreten eines vordefinierbaren Ereignisses ausgelöst wird.

16. Verfahren nach einem der vorstehenden Verfahrensansprüche, **dadurch gekennzeichnet, dass** das Erfassen des Desinfektionszustandes durch Betätigen eines Schaltelementes (15) erfolgt.

17. Verfahren nach einem der vorstehenden Verfahrensansprüche, **dadurch gekennzeichnet, dass** das Verfahren folgenden Verfahrensschritt umfasst:
- Ausgeben eines Aufforderungssignals, falls als Desinfektionszustand "nicht desinfiziert" für zumindest ein Segment (S) des Touchscreensensors (11) erfasst worden ist.

18. Verfahren nach einem der vorstehenden Verfahrensansprüche, **dadurch gekennzeichnet, dass** das Verfahren folgenden Verfahrensschritt umfasst:
- Erfassen einer Nutzeridentität, die jeweils spezifisch für einen Anwender des Touchscreensensors (11) ist und
- Bei Erfassen eines Nutzerwechsels: Ausgeben eines Aufforderungssignals zur Desinfizierung des Touchscreens.

## Claims

1. Disinfection monitoring unit (20) for a touch screen sensor (11) for controlling a medical device (D), in particular a dialysis device, wherein the disinfection monitoring unit (20) comprises a disinfection status sensor (21) which is configured to automatically detect the disinfection status of the touch screen sensor (11),
wherein the disinfection status sensor (21) is configured at least as a camera and as a fluid evaluation unit,
wherein the fluid evaluation unit is adapted to check for a presence of disinfectant, typically disinfecting fluid,
wherein the fluid evaluation unit is activated in a controlled manner only during a disinfection phase and not during device control,
wherein the touchscreen sensor (11) is configured to automatically recognize planar movement patterns for disinfection and to differentiate them from point or pressure operations for data input or device control, and/or
wherein the camera is configured to detect operations or contacts of the touchscreen sensor (11) and to forward them to the disinfection monitoring unit (20) for evaluation.

2. Disinfection monitoring unit (20) according to the preceding claim, **characterized in that** the detected disinfection state is represented as a state vector and identifies whether disinfection has taken place and/or that the state vector comprises a range indication that identifies the extent of disinfection and/or comprises further meta-data on disinfection.

3. Disinfection monitoring unit (20) according to any one of the preceding claims, **characterized in that** the disinfection monitoring unit (20) comprises an output unit (30) adapted to output the detected disinfection state as a disinfection signal or disinfection message.

4. Disinfection monitoring unit (20) according to one of the preceding patent claims, **characterized in that** the disinfection monitoring unit (20) is configured to detect the disinfection state via identification of temporally preceding movement patterns on the touch screen sensor (11).

5. Disinfection monitoring unit (20) according to any one of the preceding claims, **characterized in that** the disinfection monitoring unit (20) is adapted to detect the disinfection state of a plurality of predefinable segments (S) of the touch screen sensor (11).

6. Touchscreen (10) for controlling a medical device (D), in particular a dialysis device, having a touchscreen sensor (11), **characterized in that** the touchscreen (10) comprises a disinfection monitoring unit (20) according to one of the preceding patent claims directed to the disinfection monitoring unit (20).

7. Touchscreen (10) according to the preceding patent claim directed to the touchscreen (10), **characterized in that** the touchscreen (10) comprises a safety module (14) which is configured to automatically control the touchscreen (10) in a modified manner if the disinfection status sensor has detected a "non-disinfected" disinfection status by displaying all buttons, interaction areas and input fields (111, 112) in a segment (S) of the touchscreen sensor (11) detected as disinfected.

8. Touchscreen (10) according to one of the preceding patent claims directed to the touchscreen (10), **characterized in that** the touchscreen (10) further comprises a marking module (13) which is configured to visualize the detected disinfection state of the touchscreen sensor (11) or the respectively detected disinfection states of the individual segments (S) of the touchscreen sensor (11) in a spatially resolved manner on the touchscreen (10) and in particular to mark them.

9. Touchscreen (10) according to any one of the preceding claims directed to the touchscreen (10), **characterized in that** the touchscreen (10) further comprises a driver (T) for interacting the touchscreen (10) with the medical device (D), the driver (T) being driven by the data detected by a disinfection status sensor (21) for driving the touchscreen (10) depending on the disinfection status.

10. Medical device (D), in particular dialysis device, having a touch screen (10) according to at least one of the preceding patent claims directed to the touch screen (10).

11. Medical device (D) according to the immediately preceding device claim, the medical device (D) comprising a safety module (14) adapted to automatically control the touchscreen (10) in a modified manner if the disinfection status sensor (21) has detected a "non-disinfected" disinfection state by displaying all buttons, interaction areas and input fields (111, 112) in a segment (S) of the touchscreen sensor (11) detected as disinfected.

12. Method for controlling a medical device (D), in particular a dialysis device, via a touchscreen sensor (11), wherein during the control and/or operation of the medical device (D) by means of the touchscreen sensor (11), a disinfection state is automatically detected on the touchscreen sensor (11) and provided as a disinfection signal and/or disinfection message,
by evaluating touchscreen sensor contacts on the touchscreen sensor (11), and
by a fluid evaluation checks a presence of disinfectant, usually disinfection fluid,
wherein the fluid evaluation checks the presence of disinfectant only during a disinfection phase and not during device control,
wherein planar movement patterns on the touchscreen sensor (11) for disinfection are automatically recognized and differentiated from point or pressure operations for data input or device control, and/or
wherein operations or contacts of the touch screen sensor (11) are detected by means of an optical sensor and forwarded to the disinfection monitoring unit (20) for evaluation.

13. Method according to the preceding method claim, **characterized in that the method** comprises the following method step:
- Controlling a screen layout based on the detected disinfection state by displaying all input fields (112) and buttons and interaction areas (112) exclusively in an area of the touch screen sensor (11) that has been detected as disinfected.

14. Method according to one of the preceding method claims, **characterized in that** the evaluation of the touchscreen sensor contacts on the touchscreen sensor (11) is carried out by identifying planar movements, in particular by an optical sensor.

15. Method according to one of the preceding method claims, **characterized in that** the detection of the disinfection state can be activated and deactivated by detecting a confirmation signal or is automatically triggered after the occurrence of a predefinable event.

16. Method according to one of the preceding method claims, **characterized in that the** disinfection state is detected by actuating a switching element (15).

17. Method according to any of the preceding method claims, **characterized in that the method** comprises the following method step:
- Outputting a prompt signal if "not disinfected" has been detected as the disinfection state for at least one segment (S) of the touchscreen sensor (11).

18. Method according to any of the preceding method claims, **characterized in that the method** comprises the following method step:
- capturing a user identity, each of which is specific to a user of the touchscreen sensor (11); and
- When a user change is detected: Issue a prompt signal to disinfect the touchscreen.

## Revendications

1. Unité de surveillance de désinfection (20) pour un capteur à écran tactile (11) pour la commande d'un appareil médical (D), en particulier un appareil de dialyse, l'unité de surveillance de désinfection (20) comprenant un capteur d'état de désinfection (21) qui est conçu pour détecter automatiquement l'état de désinfection du capteur à écran tactile (11),
le capteur d'état de désinfection (21) étant conçu au moins comme une caméra et comme une unité d'évaluation de fluide,
dans lequel l'unité d'évaluation de fluide est conçue pour vérifier une présence de désinfectant, en général de liquide de désinfection,
dans lequel l'unité d'évaluation de fluide est activée de manière commandée uniquement pendant une phase de désinfection et non pendant la commande de l'appareil,
le capteur à écran tactile (11) étant conçu pour reconnaître automatiquement des modèles de mouvements plats pour la désinfection et pour les différencier de commandes ponctuelles ou par pression pour l'entrée de données ou pour la commande de l'appareil, et/ou
la caméra étant conçue pour détecter des manipulations ou des contacts du capteur d'écran tactile (11) et les transmettre à l'unité de surveillance de désinfection (20) pour évaluation.

2. Unité de surveillance de désinfection (20) selon la revendication précédente, **caractérisée en ce que** l'état de désinfection détecté est représenté sous la forme d'un vecteur d'état et caractérise si une désinfection a eu lieu et/ou **en ce que** le vecteur d'état comprend une indication de zone qui caractérise l'étendue de la désinfection et/ou comprend d'autres métadonnées concernant la désinfection.

3. Unité de surveillance de désinfection (20) selon l'une des revendications précédentes, **caractérisée en ce que** l'unité de surveillance de désinfection (20) comprend une unité de sortie (30) qui est conçue pour émettre l'état de désinfection détecté sous forme de signal de désinfection ou de message de désinfection.

4. Unité de surveillance de désinfection (20) selon l'une des revendications précédentes, **caractérisée en ce que** l'unité de surveillance de désinfection (20) est conçue pour détecter l'état de désinfection par l'intermédiaire d'une identification de modèles de mouvement précédents dans le temps sur le capteur à écran tactile (11).

5. Unité de surveillance de désinfection (20) selon l'une des revendications précédentes, **caractérisée en ce que** l'unité de surveillance de désinfection (20) est conçue pour détecter l'état de désinfection de plusieurs segments prédéfinissables (S) du capteur à écran tactile (11).

6. Ecran tactile (10) pour la commande d'un appareil médical (D), en particulier d'un appareil de dialyse, avec un capteur d'écran tactile (11), **caractérisé en ce que** l'écran tactile (10) comprend une unité de surveillance de désinfection (20) selon l'une des revendications précédentes, orientée vers l'unité de surveillance de désinfection (20).

7. Ecran tactile (10) selon la revendication précédente, visant l'écran tactile (10), **caractérisé en ce que** l'écran tactile (10) comprend un module de sécurité (14) qui est conçu pour commander l'écran tactile (10) de manière automatiquement modifiée dans le cas où le capteur d'état de désinfection a détecté un état de désinfection "non désinfecté", en représentant toutes les surfaces de commutation et d'interaction et les champs de saisie (111, 112) dans un segment (S) du capteur d'écran tactile (11) détecté comme désinfecté.

8. Ecran tactile (10) selon l'une des revendications de brevet précédentes, dirigées vers l'écran tactile (10), **caractérisé en ce que** l'écran tactile (10) comprend en outre un module de marquage (13) qui est conçu pour visualiser et en particulier pour marquer sur l'écran tactile (10), avec une résolution spatiale, l'état de désinfection détecté du capteur d'écran tactile (11) ou les états de désinfection respectivement détectés des différents segments (S) du capteur d'écran tactile (11).

9. Ecran tactile (10) selon l'une des revendications précédentes, dirigées vers l'écran tactile (10), **caractérisé en ce que** l'écran tactile (10) comprend en outre un pilote (T) pour l'interaction de l'écran tactile (10) avec l'appareil médical (D), qui est commandé par les données déterminées par un capteur d'état de désinfection (21) pour la commande de l'écran tactile (10) en fonction de l'état de désinfection.

10. Appareil médical (D), en particulier appareil de dialyse, avec un écran tactile (10) selon au moins l'une des revendications de brevet précédentes dirigées vers l'écran tactile (10).

11. Appareil médical (D) selon la revendication d'appareil immédiatement précédente, l'appareil médical (D) comprenant un module de sécurité (14) qui est conçu pour commander l'écran tactile (10) de manière automatiquement modifiée dans le cas où le capteur d'état de désinfection (21) a détecté un état de désinfection "non désinfecté", en représentant toutes les surfaces de commutation et d'interaction et tous les champs de saisie (111, 112) dans un segment (S) du capteur d'écran tactile (11) détecté comme désinfecté.

12. Procédé de commande d'un appareil médical (D), notamment d'un appareil de dialyse, par l'intermédiaire d'un capteur à écran tactile (11), dans lequel, pendant la commande et/ou le fonctionnement de l'appareil médical (D) au moyen du capteur à écran tactile (11), un état de désinfection est détecté automatiquement sur le capteur à écran tactile (11) et est mis à disposition sous forme de signal de désinfection et/ou de message de désinfection,
en évaluant les contacts de l'écran tactile sur le capteur de l'écran tactile (11), et
en vérifiant par une évaluation du fluide une présence de désinfectant, en général de liquide de désinfection,
l'évaluation du fluide ne vérifiant la présence de désinfectant que pendant une phase de désinfection et non pendant la commande de l'appareil,
des modèles de mouvements plats sur le capteur à écran tactile (11) étant automatiquement reconnus pour la désinfection et différenciés des commandes ponctuelles ou par pression pour l'entrée de données ou la commande de l'appareil, et/ou
les manipulations ou les contacts du capteur d'écran tactile (11) étant détectés au moyen d'un capteur optique et transmis à l'unité de surveillance de désinfection (20) pour évaluation.

13. Procédé selon la revendication de procédé précédente, **caractérisé en ce que** le procédé comprend l'étape de procédé suivante :
- commander une structure d'écran sur la base de l'état de désinfection détecté, **en ce que** tous les champs de saisie (112) et les surfaces de commutation et d'interaction (112) sont représentés exclusivement dans une zone du capteur d'écran tactile (11) qui a été détectée comme désinfectée.

14. Procédé selon l'une des revendications de procédé précédentes, **caractérisé en ce que** l'évaluation des contacts de capteur d'écran tactile sur le capteur d'écran tactile (11) est effectuée en identifiant des mouvements de surface, en particulier par un capteur optique.

15. Procédé selon l'une des revendications de procédé précédentes, **caractérisé en ce que** la détection de l'état de désinfection peut être activée et désactivée par la détection d'un signal de confirmation ou est déclenchée automatiquement après l'apparition d'un événement prédéfinissable.

16. Procédé selon l'une des revendications de procédé précédentes, **caractérisé en ce que** la détection de l'état de désinfection s'effectue par l'actionnement d'un élément de commutation (15).

17. Procédé selon l'une quelconque des revendications de procédé précédentes, **caractérisé en ce que** le procédé comprend l'étape de procédé suivante :
- émettre un signal d'invitation si l'état de désinfection "non désinfecté" a été détecté pour au moins un segment (S) du capteur à écran tactile (11).

18. Procédé selon l'une quelconque des revendications de procédé précédentes, **caractérisé en ce que** le procédé comprend l'étape de procédé suivante :
- l'acquisition d'une identité d'utilisateur, dont chacune est spécifique à un utilisateur du capteur d'écran tactile (11), et
- Lorsqu'un changement d'utilisateur est détecté : émission d'un signal d'invitation à désinfecter l'écran tactile.
